# EUROPEAN PATENT APPLICATION

(11) **EP 4 226 858 A1**
(43) Date of publication of application: **16.08.2023**
(21) Application number: 22155767.1
(22) Date of filing: 09.02.2022
(51) Int. Cl.: A61B 5/08, A61B 5/16, A61B 5/00, A61B 5/0205, A61B 5/024

(54) **BIOFEEDBACK SYSTEM**

(71) Applicant: Neubit Oy, 00650 Helsinki (FI)
(72) Inventor: HOUGHTON, Paul, 02600 Helsinki (FI); REPONEN, Marko, 00650 Helsinki (FI); ZUBAREV, Ivan, 02650 Espoo (FI); MÄÄTTÄ, Kalle, 00560 Espoo (FI); VIRTA, Otto, 00270 Helsinki (FI); LINDSTRÖM, Ville, 00560 Helsinki (FI)
(74) Representative: Berggren Oy

(57) **Abstract**

The invention relates to a biofeedback training system aimed to 1) detect and estimate a physiological state in the human subject, 2) provide the information about such state back to the subject, and 3) provide a set of dynamic instructions to the subject aimed at voluntarily changing the physiological state towards the desired state, defined by the goal of the training session.

## Description

### Technical field

The application relates to a system for acquiring physical data related to a physiological state from a human subject and communicating it back to the subject in the form, allowing the subject to become aware of their physiological state in real time..

### Background

US2020/0288978A1 discloses methods and systems provided to monitor biosignals associated with one or more physiological structures of a subject. It processes biosignals to determine neural information and uses the neural information to determine current state of the subject, determines feedback to be provided to the subject based on the current state and a desired state, and/or provides the feedback to the subject in order to modify the subject's current state to the desired state.

Stress is a non-specific physiological reaction to disturbing, unpredictable, or uncontrollable events. In response to such stressors the hypothalamo-pituitary-adrenal system (HPA) releases stress hormones (mainly, adrenaline and cortisol) activating the sympathetic branch of the autonomic nervous system (ANS). Once the stressful situation is over, the parasympathetic branch of the ANS dampens the initial stress response returning the organism back to low-arousal state, a process known as "stress resilience". Chronic exposure to stress, however, is known to decrease stress-resilience. Leading to numerous adverse psychological (i.e. burnout, anxiety, depression etc.) and physiological (i.e. hypertension, cardio vascular disease, immune system suppression etc) effects. Moreover, absence of efficient stress-coping strategies often leads to indirect problems stemming from maladaptive 'displacement behaviours' to reduce stress such as substance abuse, eating disorders, etc.

US2020/0288978A1 states that the feedback is acoustic stimuli, such as human speech, singing, instrumental music or synthesized music, which may be synchronized with visual stimuli.

Learning efficient stress-coping strategies can contribute to the prevention of stress-related disorders. However, learning to cope with stress requires a high-level of emotional awareness and can be extremely challenging without external guidance. Indeed, numerous relaxation techniques exist, but it is extremely hard to choose an appropriate technique for each individual without any external feedback.

### Summary

It is an object of the disclosure to provide an improved system, and to alleviate at least some of the problems associated with biofeedback systems of the prior art.

The object of the disclosure efficiently combines biofeedback with guidance signals when performing biofeedback training in order to reduce stress or otherwise change the physiological state of the subject. Such combination is missing from the prior art.

The present disclosure describes a directional biofeedback that is intended to facilitate the natural physiological recovery mechanisms of humans. Any of the used biofeedback signals may be used to modulate the guidance signals or presented separately along the biofeedback signals to achieve the desired state of the given measured biosignal more efficiently.

A biofeedback system comprises a biosignal acquisition unit, a pre-processing unit, a processing unit, and a biofeedback unit. The biosignal acquisition unit may be a sensor arrangement to receive, amplify, and transmit at least one biosignal. The pre-processing unit may be integrated to the processing unit. The processing unit comprises at least one processor and a storage for at least one database, a biographical and historical database for subject data including at least the at least one biosignal.

The biosignal acquisition unit is configured to measure at least one biosignal from a subject. The pre-processing unit is configured to remove artifacts, and/or increase the signal-to-noise ratio of the at least one biosignal. The at least one processor of the processing unit is configured to extract features from the at least one biosignal, estimate an individual physiological state of the subject, define target state of the subject, calculate an instruction signal of the subject, and determine a guidance signal.

Estimation of an individual physiological state of the subject is based on the extracted features, including at least one extracted ANS parameter, the biographical and historical database based dynamic baseline information calculated using a predictive model, and stress level estimation calculated from the ANS parameters and the dynamic baseline information. The defined target state is calculated from stress level estimation, dynamic baseline information of the subject and subject given input. The instruction signal is calculated based on the stress level estimation of the individual physiological state of the subject. The guidance signal is determined based on the instruction signal and the defined target state of the subject.

The biofeedback unit is configured to deliver to the subject physiological state-dependent biofeedback according to the guidance signal. The guidance signal is based on the calculated instruction signal. The instruction signal may be the guidance signal. The biofeedback is arranged for guidance of the direction, depth, and/or frequency of the breathing of the subject.

The biofeedback unit compares the individual physiological state before the delivered guidance signal with the individual physiological state after the delivered guidance signal. Then the biofeedback unit adjust feedback parameters of the instruction signal and guidance signal such that the feedback outcome approaches the defined target state, and finally updates the biographical and historical database with current values

According to an embodiment, the biosignals may be related to the state of the autonomous nervous system of the subject.

According to an embodiment, the biofeedback may be arranged for haptic guidance of vertical spatial haptic signals instructing inhale and exhale duration and horizontal spatial haptic signals instructing inhale and exhale hold duration.

According to an embodiment, the guidance signal may include complex multichannel audio-haptic stimulation using low frequency sound waves, their harmonics, interference patterns, and relative phase-amplitude modulations corresponding to the perceived effect of their slow (2-12 cm/s) spatial propagation in vertical and horizontal directions.

According to an embodiment, the biographical and historical database may comprise biographical information, previous measurement history, previous biofeedback history, population level-statistics, circadian data, and/or one or more sources of individual physiological variation.

According to an embodiment, the biographical and historical database may comprise at least one or more sources of individual physiological variation, and optionally biographical information, previous measurement history, previous biofeedback history, population level-statistics, circadian data.

According to an embodiment, the biofeedback may include audio guidance as audible audio stimulus or feedback based on the instruction signal and the defined target state.

According to an embodiment, the biofeedback system may be arranged into a seating arrangement.

According to an embodiment, the biofeedback system may be arranged into a seating arrangement, and contact points between a subject and a biosignal acquisition unit may be arranged on armrests of the seating arrangement. The contact points between the subject and the biosignal acquisition unit may be arranged to receive the biosignals from forearm area of the subject, and reference and grounding from elbow area of both arms of the subject.

According to an embodiment, the biofeedback system may be arranged into a seating arrangement with contact points between a subject and a biosignal acquisition unit arranged in a backrest of the seating arrangement.

A biofeedback method comprises measuring at least one biosignal from a subject, pre-processing the at least one biosignal to remove artifacts, and/or increase the signal-to-noise ratio of the at least one biosignal, storing the at least one biosignal, arranging a biographical and historical database for the at least one biosignal, extracting features from the at least one biosignal, estimating individual physiological state of the subject based on the feature extraction and the biographical and historical database, calculating an instruction signal based on the estimated individual physiological state, providing to the subject a guidance signal of vibrotactile biofeedback according to the instruction signal and a defined target state to arrange biofeedback for breathing guidance.

The biofeedback method further comprises comparing the individual physiological state before the delivered guidance signal with the individual physiological state after the delivered guidance signal, adjusting feedback parameters of the instruction signal and the guidance signal such that the feedback outcome approaches the defined target state, and updating the biographical and historical database with current values.

According to an embodiment, calculating an instruction signal in the biofeedback method may include calculating a ratio between current stress level estimation of at least one biosignal, and a dynamic baseline information based on a predictive model calculated from biographical and historical database.

What is disclosed is described in the independent claims. Other embodiments are described in dependent claims.

### Brief description of the drawings

Next the solution will be described in greater detail with reference to the accompanying drawings, in which:
Figure 1 is an embodiment of a biofeedback process flowchart.
Figure 2 shows at 2A a box-plot demonstrating single biofeedback session induced change as self-reported mood and agitation, while 2B is a box-plot demonstrating physical change of heart beat related activities during the same single biofeedback session.
Figure 3 is a flowchart demonstrating the computational steps in order to extract physiologically-relevant parameters related to individual's stress level from the ECG measurements.
Figure 4 is a flowchart demonstrating the steps in order to deliver appropriate biofeedback to the subject.
Figure 5 is a flowchart demonstrating the modulation of the breathing instructions by a biofeedback signal.

The figures are schematic. The figures are not in any particular scale.

### Detailed description

The solution is described in the following in more detail with reference to some embodiments, which shall not be regarded as limiting.

In this description and claims, term "comprising" may be used as an open term, but it also comprises the closed term "consisting of".

Within context of this disclosure, heart activity measured from any location of the subject's body may be called electrocardiogram (ECG). ECG may be called biosignal. Information extracted from physical measurements may be called extracted features. The extracted features may be called physiologically relevant features. Further, the extracted features may be called estimates of the physiological state of the subject. The physiological state of the subject may be referred to as physiological stress. Biofeedback may be referred to as a set of signals communicating the level of physiological stress back to the human subject. Therefore, biofeedback signal may refer to an instruction signal that is generated in order to provide, for example, detailed breathing instructions. Biofeedback signal may also refer to a guidance signal that is constructed according to the instruction signal and provided to the subject describing how their behavior affects their physiological state, such as stress level, measured by their HRV. In essence, guidance signals may be referred to as a set of instructions provided to the human subject aimed at modifying their behaviour (e.g. depth and the duration of their breathing) voluntarily.

Biofeedback signal may further refer to the verbal or other guidance provided to the subject to better understand the interrelations between subject's breathing and physiology.

Within context of this disclosure, the human subject may be called a user or a subject, when applicable.

Within context of this disclosure, the vibrotactile feedback may be called haptic feedback.

Within context of this disclosure, BREATH may be used as an acronym for biofeedback seating arrangement experiments using a biofeedback system of the disclosure.

Biological feedback or biofeedback uses objective measurements of the subject's physiological state and provides the information extracted from these measurements back to the subject in an intuitively understandable form. The subject may then use this information to gain awareness and some degree of control over their physiological state in order to learn e.g., self-regulation or stress coping techniques. Biofeedback is typically complemented by a learning task, such as a guided breathing exercises aimed to achieve the desired physiological state. In the present disclosure, biofeedback provides the subject with information about their progress.

Since stress is associated with notable increases sympathetic nervous system activity, it can thus be inferred though non-invasive proxy measures such as increased heart rate, heart rate variability (HRV), skin conductance and locomotion parameters indicating autonomic arousal and reactivity. It is obvious to a person skilled in the art that such non-invasive proxy measures may be used interchangeably or in combinations in different realizations of the disclosed biofeedback system.

HRV provides an estimate of the interplay between the inputs from the sympathetic and the parasympathetic branches of the ANS onto the cardiac activity and can be used as an indication of physiological stress level.

According to an embodiment autonomic nervous system activity of a subject may be recorded while the subject is comfortably seated on a seating arrangement, such as a chair. The seating arrangement may include a seat part, a backrest part, and separate armrests for left and right arm.

According to an embodiment biosignal, such as electrocardiogram (ECG), may be measured from contact points arranged to enable biosignal acquisition from a subject. For example, ECG may be measured from finger, wrist, forearm or chest. ECG may be converted into a digital form. Thereafter, ECG measurements may be transformed using a number of pre-processing and processing steps to reduce the measurement noise, to estimate signal-to-noise ratio (SNR), and to estimate the measurement quality in real time. The steps may include, but are not limited to, de-noising, amplification, filtering, error correction, outlier removal, and resampling. The filtering may be lowpass, band-pass, notch, and/or high-pass filtering. The pre-processed signal of sufficient quality may be used to detect individual cardiac events of interest, such as QRS complex, T-wave, and respiratory sinus arrythmia. Time domain and frequency-domain features relevant to estimating various components and measures of the heart-rate variability (HRV) may be extracted from the pre-processed signal.

### Signal processing steps

Linear trend may be removed from the ECG signal by fitting and subtracting 1^{st} order polynomial. The detrended ECG data may be filtered in 1-100 HZ, preferably 2-40Hz pass-band. Signal-to-noise ratio (SNR) may be estimated by computing the variance ratio between the two sliding windows with duration of 20-2000 ms, preferably 40-80 ms, more preferably 60 ms (signal variance) and 1000 ms (noise variance). For each segment where SNR exceeds 0.9, r-wave peaks may be obtained by finding the maximum signal value within the adaptive amplitude bounds, defined as 55% and 175% of averaged amplitude of 3 - 10 preferably 5 previously detected peaks, adjusted by the gaussian likelihood of observing a peak given the previous estimates of the heart rate and the heart-rate variability (HRV). Amplitude bounds, heart-rate and HRV estimates may be updated after each successful detection.

### HRV estimation and extracted features

Intervals between the successive peaks, detected by the previous step (R-R intervals), may be used to obtain time-domain (e.g. SDNN, RMSSTD, PP50) and frequency domain (e.g. power-spectral density of the tachogram interpolated to equidistant sampling rate, LF-HRV, HF-HRV, dominant frequency and it's harmonics, coherence to the frequency of the instruction signal frequency) features of the heart-rate variability.

### Estimation of the individual physiological state

According to an embodiment, the features extracted from the biosignal data may be combined with subject's biographical information, previous measurement history, as well as population level statistics and other relevant sources of physiological variation. Sources of physiological variation may include, but are not limited to, individual circadian variations, body mass index, cardiac fitness level, measured circulating hormonal levels from saliva or blood, age, and/or, gender. These sources of physiological variation may be used in combination with biosignal measurements to infer individual's level of physiological stress using a predictive generative model.

Estimates of HRV are compared to the baseline HRV parameters derived from the predictive machine-learning model comprising the subject's biographic data (age, seg, body mass index), individual circadian rhythm, and history of the previous measurements to obtain the estimation of the level of physiological stress. Stress index, defined as the directional statistical deviation from the expected parameters derived from the predictive model is used to define the target state and provide biofeedback signal, which can be provided as a separate signal or used to modulate the guidance signals.

### Guidance and biofeedback signals

According to an embodiment, the extracted features may be transformed into a biofeedback signal designed to provide information about the subject's physiological state back to the subject and/or provide guidance during training sessions. The goal of such sessions may be, for example, to reduce the level of physiological stress and/or train stress coping skills. The goal may be reached, for example, by breathing intentionally slowly at a pre-determined pattern of inhale and exhale duration.

According to an embodiment audio-haptic signals guide the human subject to perform various tasks such as deep breathing exercises. The guidance signal can include voice instructions, music, and vibrotactile patterns with varying spatial, temporal, and spectral properties or a combination thereof. The guidance signal may comprise speech, music, and a combination of multichannel low-frequency (20-200 Hz) soundwaves, their harmonics, reverberations, interference patterns, as well as alterations of their relative phases and amplitudes, corresponding to a perceived effect of their spatial propagation in vertical and/or horizontal directions.

According to an embodiment, the system can be designed such that the instruction signal is the guidance signal and no further conversions or calculations between the signals is required.

According to an embodiment the biofeedback may be directional. The directionality may instruct the subject to alter the duration and/or the direction of the breathing according to the biofeedback instructions. The instructions may be tactile and/or audio biofeedback which guide the subject to breathe in and breathe out according to the rhythm provided by the tactile and/or audio stimuli. Haptic guidance signals propagating in the upward direction instruct the subject to inhale and haptic guidance signals propagating in the downward direction instruct the subject to exhale. Other haptic guidance signals may instruct the subject to hold their breath following the inhale or exhale.

According to the embodiment continuous real-time audio-haptic biofeedback signal indicates the user's current physiological state back to the user. The biofeedback can be presented as a separate audio-haptic signal or by modulating the parameters (e.g. volume, amplitude, frequency, duration etc.) of the guidance signals. According to the embodiment, the biofeedback signal is derived from the measurements of autonomic modulations of the cardiac activity as measured by the ECG. The biofeedback signal may include phase, amplitude or frequency modulations of the guidance signal, emergence and/or disappearance of the distinct components of the guidance signal or represent a distinct biofeedback audio, haptic, or visual signal or combination thereof.

According to an embodiment guidance and biofeedback signals relate to each other in such a way that the guidance signal instructs the user to perform a task typically aimed at regulating the physiological state. The biofeedback signal indicates the degree of success in performing such task back to the user

The user experience may be designed in a way that the subject learns to interpret the biofeedback intuitively or requiring only brief initial instructions and can be performed without the presence of a trained professional.

According to an embodiment, the directionality of the breathing guidance may include tactile and/or audio guidance that, for example, induce vibrations that move vertically along a backrest of a seating arrangement from the bottom of the backrest to the top of the backrest resembling the desired inhalation or in-breathing. The directionality of the breathing guidance may include tactile and/or audio guidance that, for example, induce vibrations that move vertically along the backrest of the seating arrangement from the top of the backrest to the bottom of the backrest resembling the desired exhalation or out-breathing. The directionality guidance may include horizontal breathing guidance. The directionality guidance may include horizontal and vertical breathing guidance.

The biofeedback system run sessions may be repeated as desired or as required. For example, the sessions can be repeated at predefined time intervals or predefined number of times. In addition, sessions may be repeated based on subject needs or, for example, as long as the desired stress coping skills or stress reduction has been achieved.

According to an embodiment, subjects undergoing rehabilitation from alcohol use disorder may benefit from the biofeedback system. The biofeedback system may be used to seek and to identify physiological signatures of high stress level following the exposure to visual alcohol-related cues. Thereafter, the biofeedback system may be used to reduce stress using a combination of biofeedback and other guided exercises. The other guided exercises may include additional breathing exercises.

According to an embodiment, the biofeedback system may be used to train subjects to cope with workplace stress and/or to improve recovery from stress. The biofeedback system may further be used in other forms of psychotherapy and/or rehabilitation. It is obvious for a person skilled in the art that the biofeedback system may be used generally to individually train subjects to observe, control and cope with stress-related neurological and psychiatric disorders.

According to an embodiment, biofeedback system may be used as a multi-session biofeedback training system to instruct healthy subjects to realize the relationships between the resting state heart rate, heart rate variability, breathing patterns, and self-reported stress levels.

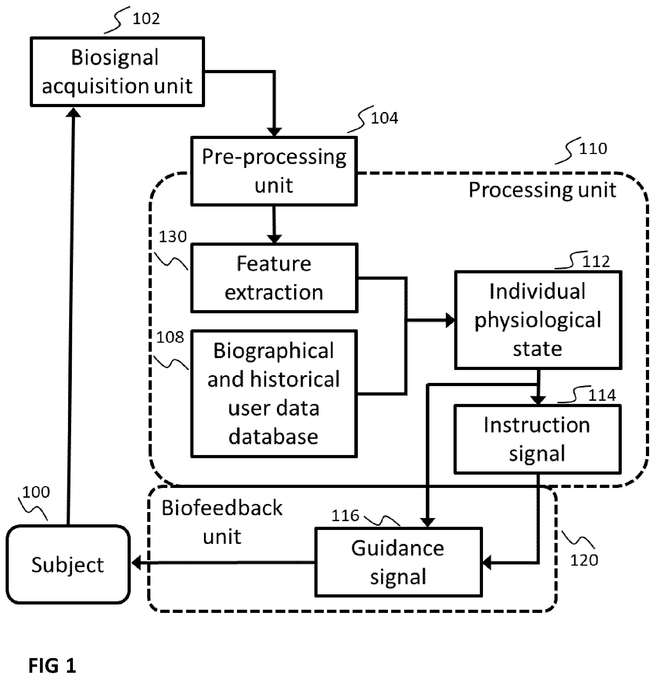

Figure 1 is an embodiment of a biofeedback process flowchart. According to figure 1, biosignal related to the autonomic nervous system activity may be acquired from the subject 100. A biosignal acquisition unit 102 may be arranged for receiving biosignals from the subject 100. The biosignal acquisition unit 102 may comprise one or more sensors, electrodes, or other biosignal receiving arrangements. The biosignal acquisition may be continuous, intermittent, or sample-and-hold acquisition. The biosignal acquisition unit 102 may include one or more amplifiers, rectifiers and/or filters. Pre-processing unit 104 may be integrated to the biosignal acquisition unit 102. The acquired biosignal be may be an electrocardiogram (ECG). The acquired biosignal may be converted into a digital signal.

The digital biosignal may be pre-processed 104 in order to, for example, improve signal-to-noise ratio and to remove artefacts arising from for example movements, loss of electrode to skin contact or other sources of signal contamination such as electric activity of the muscles at the measurement site.The pre-processing unit 104 may be a separate unit or it may be integrated into a processing unit 110. Processing unit 110 may be configured to have a storage and/or a memory. The processing unit 110 may be further arranged to have a database. The processing unit may be further arranged to have at least one processor for computational tasks including, but not limited to, feature extraction 130, estimation of an individual physiological state 112, and calculation of an instruction signal 114 . The instruction signal 114 may be used to calculate a guidance signal 116 to inform the subject how to adjust their behavior, such as for example, duration of the inhale or the exhale of breathing. Biofeedback based on guidance signal 116 may be used to inform the subject the degree of success in performing the breathing task. Biofeedback may include information about how the behavior of the subject affects the physiological state, such as stress level, of the subject, for example measured by their HRV.

Features relevant to the physiological state of the subject may be extracted 130 from the pre-processed biosignal. For example, heart rate variability (HRV) may be extracted from the pre-processed biosignal. Personal subject 100 information may be stored in a biographical and historical user data database 108. Accuracy of the estimation of the physiological state may be improved by including the biographical data and historical user information 108 to the biofeedback system. The individual physiological state 112 may be estimated from the biosignal feature extraction 130 together with the biographical and historical user data database 108 information. The individual physiological state 112 may include a stress index calculated based on HRV. The guidance signal 116 for delivering the physiological state dependent biofeedback to the subject 100 is calculated based on the instruction signal 114. The instruction signal may be calculated from the estimated individual physiological state 112 of the subject 100. The guidance signal 116 may additionally be based on an algorithm that is programmed to attempt to increase HRV value of the subject 100.

A biofeedback unit 120 may be used to deliver the physiological state dependent guidance to the subject 100. The instruction signal 114 may comprise instructions for guidance signal 116 that is arranged to guide the direction, depth, and/or frequency of the breathing of the subject 100. The vibrotactile guidance may be designed to increase parasympathetic nervous system activity. The vibrotactile guidance may provide specific breathing instructions including, for example, biofeedback unit specific instructions comprising the required frequency and amplitude of the feedback to deliver desired physiological state dependent biofeedback. Additional audible guidance may be provided together or intermittently with the vibrotactile guidance. The additional audible guidance may be synchronized with the vibrotactile guidance. The audible guidance may be spatial sound. Additional guidance reflecting the physiological relationship between stress and breathing may be provided with the vibrotactile guidance. Estimation of the individual physiological state 112 of the subject 100 may include estimation of individual level of stress.

The individual physiological state 112 of the subject before the delivered guidance signal 116 is compared with the individual physiological state 112 determined after the delivered guidance signal 116. The comparison of the individual physiological state 112 of the subject before the delivered guidance signal 116 with the individual physiological state 112 determined after the delivered guidance signal 116 may be based on continuous acquisition or periodical acquisition with even or uneven intervals. The acquisition may include averaging of successive guidance signals.

According to an embodiment, duration of the biosignal acquisition may be the sampling frequency of the biosignal acquisition unit 102. The duration of the biosignal acquisition may be adapted for optimal sampling frequency of one or more autonomic nervous system parameters. The duration of the biosignal acquisition may be one or more breathing cycles. Duration of the guidance signal 116 may be the sampling frequency of the biofeedback unit 120. Duration of the guidance signal 116 may be adapted for the optimal sampling frequency of one or more autonomic nervous system parameters. Duration of the guidance signal 116 may be one or more breathing cycles.

The biofeedback unit 120 may be a dynamically adjusting user interface. The biofeedback unit 120 may be integrated into the processing unit 110. The biofeedback unit 120 may be used to dynamically adjust the biofeedback and/or guidance based on the outcome of the previous vibrotactile guidance. The outcome may be estimated as changes in the physiologically relevant parameters of the measured physical biosignals. In other words, The biofeedback unit 120 may be used to update the database 108 based on feature extraction 130, vibrotactile guidance signal 116, and the preceding values of the database 108. This may allow the biofeedback to dynamically adjust to the incoming biosignals. In essence, The biofeedback unit 120 may estimate the effect of the vibrotactile biofeedback by comparing the individual physiological state 112 before and after delivering a guidance signal 116 based feedback to the subject 100. The dynamically adjusting biofeedback may be programmable to optimize guidance signals in order to increase HRV value or reduce stress of the subject 100. The dynamically adjusting biofeedback may provide the subject 100 vibrotactile breathing guidance in order to reduce or cope with stress.

According to an embodiment the physiological state 112 may include estimation of a stress level. The physiological state 112, the instruction signal 114, and thereby the guidance signal 116 for the vibrotactile biofeedback, may be calculated one breath at the time, i.e. individually for each inhalation and exhalation.

According to an embodiment the biofeedback system may be integrated into a seating arrangement. The seating arrangement may include armrests and a backrest. The contact points may be arranged on the armrests of the seating arrangement such that the biosignals may be measured from forearm area of the subject 100 against the reference and grounding from elbow area of the subject 100. The contact points may be part of the biosignal acquisition unit 102. The biosignal may be measured from both forearms (Einthoven lead I axis) using dry electrodes integrated into the armrests of the seating arrangement with grounding and reference electrodes located under both elbows. The biosignals may be measured from the forearm area using 1-10 electrodes, preferably 4 electrodes. The grounding electrode may be on the elbow area of the subject. Grounding electrode and reference electrode may be the same electrode. Grounding electrode and reference electrode may be separate electrodes. The processing unit 110 may be positioned below the seat or on the side of the backrest that is facing away from the subject 100. The vibrotactile unit may be positioned on the side of the backrest that is facing the subject 100.

According to an embodiment contact points between the subject 100 and the biosignal acquisition unit 102 may be arranged in the backrest of the seating arrangement. In this embodiment, the biosignal acquisition 102 unit may comprise capacitive electrodes in the chair backrest. Because there are no measuring points in the armrests in this embodiment, the embodiment allows more degrees of freedom for the movements of the subject 100, even during the measurement.

According to an embodiment the biosignal acquisition unit 102 of the biofeedback system may comprise at least capacitive electrodes as sensor elements. Using capacitive electrodes introduces an additional advantage because it makes it possible that the contact points do not need to be in direct skin contact with the subject. This provides the benefit of reducing preparation time and increasing convenience when using the system.

According to an embodiment the biofeedback system may be an additional element of the seating arrangement. For example, the biofeedback system may be a sheet-like cover that is arrangeable on top of the seating arrangement.

According to an embodiment the biofeedback system or parts of the biofeedback system, such as one or more units of the biofeedback system, may be arranged into a horizontal surface that enables a contact between the biosignal acquisition unit and a subject. Specifically, the biofeedback system may be arranged in a bed or a mattress. Having the biofeedback system in a mattress may enable a more relaxed measuring, training or therapy position for the subject. In addition, incorporating the biofeedback system in a mattress may facilitate in performing longer, even overnight, measurement, training or therapy sessions.

It is obvious to a person skilled in the art that, if necessary, the biofeedback system may also be arranged to any vertical or angled surface that enables a contact between the biosignal acquisition unit of the biofeedback system and the subject.

According to an embodiment the biofeedback system may be a wearable system. The biosignal acquisition unit 102 may be a wearable ankle, arm, chest, back or wrist-worn device. The biofeedback unit 120 may be a bodice or a harness that has the vibrotactile elements facing the subject on the back side of the bodice or the harness. The pre-processing unit 104 and the processing unit 110 may be arranged to the biosignal acquisition unit 102 and/or The biofeedback unit 120.

According to an embodiment the biofeedback unit 120 may have 1-20 channels, preferably 8-10 channels, to deliver the biofeedback. Each channel of each of the sources may be separately operable. The channels may have 1-4 sources, preferably 3 sources, for example music, audio guidance, and vibration. One or more of the channels may be audible audio channels. Audible audio channels may be arranged to deliver music and/or audio guidance.

Biofeedback may be a combination of music, audio guidance, and vibration. In a preferred embodiment biofeedback has 2-4 channels for the audio and 4-18 channels for the vibration. Vibration channels may include low frequency sound channels or other haptic or tactile source channels. The low frequency sound may be 20 -200 Hz vibrations with amplitude range of 2 - 18 m/s².

According to an embodiment, the instruction signal 114 may be used to construct a guidance signal 116. The guidance signal may include complex multichannel audio-haptic stimulation using low frequency sound waves (20-200 Hz), their harmonics, interference patterns, and relative phase-amplitude modulations corresponding to the perceived effect of their slow (2-12 cm/s) spatial propagation in vertical and horizontal directions.

All the channels and/or sources may be synchronized or linked together with defined delays and intervals. Vibrating channels may be arranged into the backrest in a matrix form. The matrix form may enable directional stimulation by sequential activation of the vibration providing channels. Different channels may be instructed to vibrate at different amplitudes and/or frequencies. Different channels may be differentially controlled by the processing unit 110. Different vibration may be delivered to different body parts. Vibration may be arranged to operate as a guidance for breathing. The guidance may involve sequential activation of the vibration providing channels in vertical and/or horizontal direction. For example, the vibration providing channels may be sequentially activated such that inhalation is instructed by channels in the matrix activating sequentially from bottom to top (vertical activation) and/or from centre to the edges (horizontal activation). Similarly, exhalation may be instructed by channels in the matrix activating sequentially from top to bottom (vertical activation) and/or from edges to centre (horizontal activation). The biofeedback by vibrotactile stimuli providing channels may provide guidance to alter the direction, depth, and frequency of each inhalation and exhalation. The change in the direction, the depth, and the frequency of each inhalation and exhalation may alter the individual physiological state 112 of the subject 100. The change in the individual physiological state 112 of the subject may be stress reduction.

According to an embodiment a stress index may describe the physiological state. The stress index may have 4-10, preferably 7, categories or be a continuous variable normalized in range from 0.0 to 1.0. The categories may be used to define the instruction signal intensity for the guidance signal of the biofeedback unit 120. The value of the continuous variable may be used to modulate the intensity of the guidance signals. The intensity of the vibration may depend on the stress index.

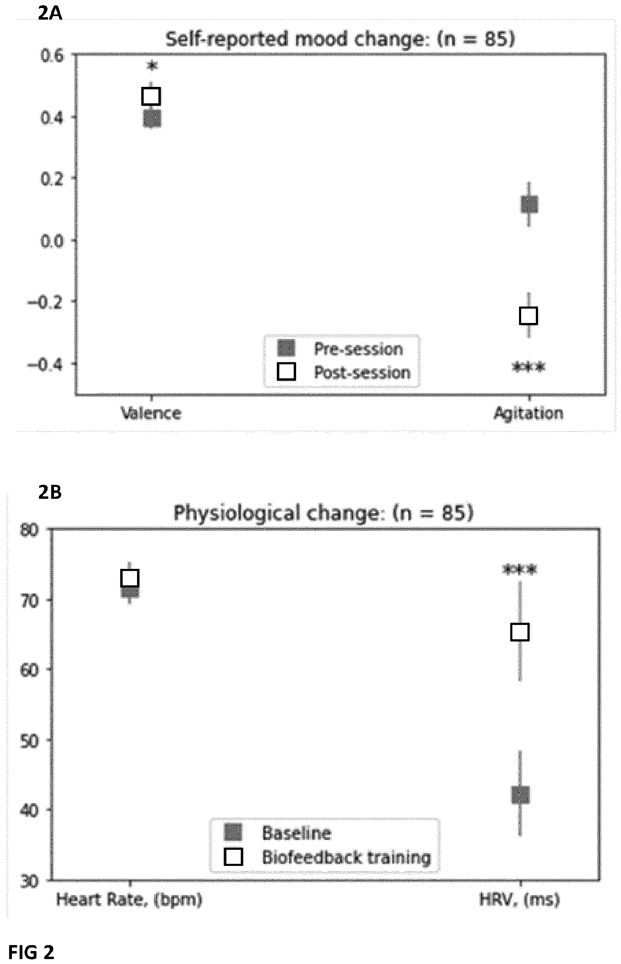

Figure 2 shows a single-session training with BREATH biofeedback system. Figure 2A demonstrates a box-plot of a single biofeedback session induced change on self-reported mood and agitation levels before (grey) and after (white) the session. Figure 2B demonstrates the physiological activity including heart-rate and heart-rate variability before (grey) and during (white) the same session (*-p < 0.05, ***-p < 0.001). The results indicate highly significant (p< 0.001, permutation test with unequal variance) effects of a single BREATH session on increase of the heart-rate variability and the decrease of the self-reported levels of agitation compared to the pre-session baseline condition in a sample (n=85) of healthy volunteers. Based on figures 2A and B it is evident that haptic breathing instructions may be used to alter the physiological state of the subject, for example to reduce stress.

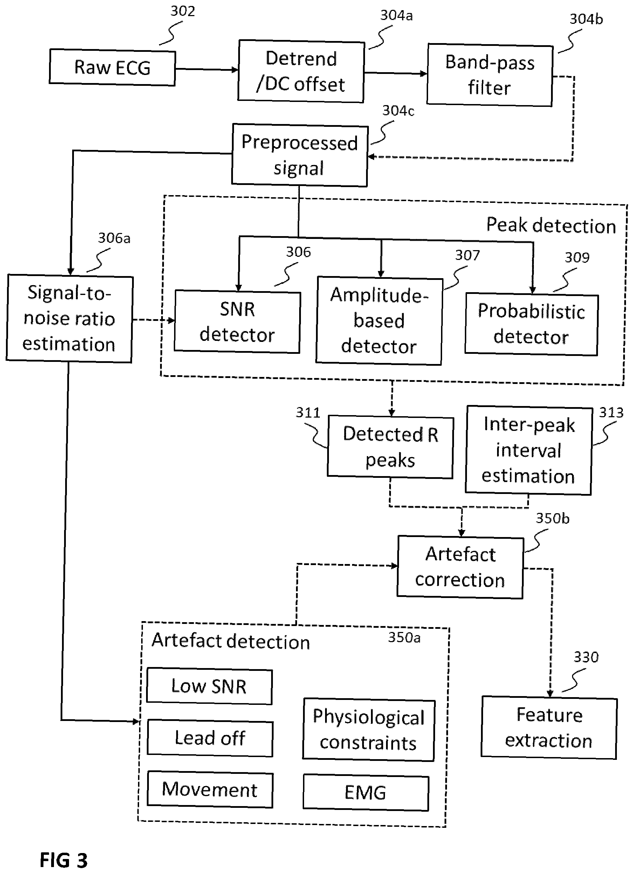

Figure 3 is a flowchart demonstrating the computational steps in order to extract physiologically-relevant parameters related to individual's stress level from the ECG measurements. Raw ECG 302 may be detrended and/or corrected for DC offset 304a and band-pass filtered 304b in order to obtain a pre-processed signal 304c. Obtaining to pre-processed signal 304c may include estimation of signal-to-noise ratio 306a. ECG peak detection may include signal-to-noise ratio detector 306, amplitude based detector 307 and/or probabilistic detector 309. Detected R peaks 311 and inter-peak interval estimation 313 may be subjected to artefact correction 350b. Artefact correction 350b may be based on artefact detection 350a. Artefact detection may be based on one or more of the following low signal-to-noise ratio, lead off, movement, physiological constraints, and electromusculogram. After artefact correction 350b of the processed signal, it is possible to obtain physiologically relevant feature extraction 330 from the measured raw ECG 302.

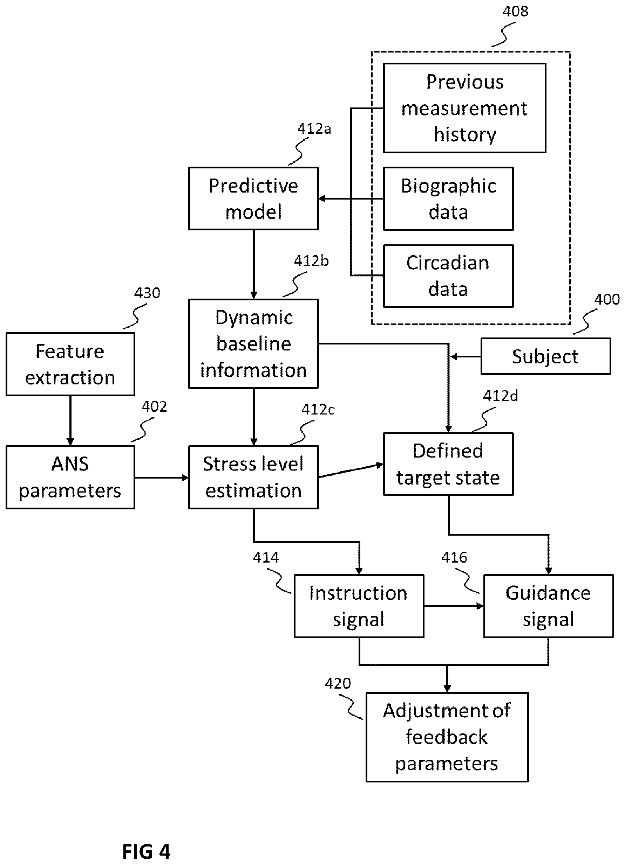

Figure 4 is a flowchart demonstrating the steps in order to deliver appropriate biofeedback to the subject. Extracted features 430 may be used to calculate autonomic nervous system (ANS) parameters 402. The ANS parameters 402 may be combined with dynamic baseline information 412b in order to gain stress level information 412c. The dynamic baseline information 412b may be based on a predictive model 412a that receives biographical and historical database information 408. The biographical and historical database information 408 may include at least one or more of the following: previous measurement history, biographic data, and circadian data. The dynamic baseline information 412b and the stress level information 412c may be supplemented with subject/user input to obtain definition of a target state 412d. The stress level estimation 412c may be used to calculate an instruction signal 414. The target state definition 412d and the instruction signal 414 may be used to construct a guidance signal 416. The instruction signal 414 and the guidance signal 416 of successive measurements may be used to determine required adjustments for the stimulation parameters 420. The guidance signal 416 communicates the current estimate of subject's physiological state as a biofeedback back to the subject optionally modulating the amplitude, frequency, or speed of spatial propagation of the instruction signal 414.

The predictive model 412a may be arranged to calculate one or more parameters related to individual subject physiology. The one or more parameters may be obtained from the biographical and historical database 408. The calculation may include comparison between recent individual subject parameter measurements against biographical information, previous measurement history, previous biofeedback history, population level-statistics, circadian data, one or more sources of individual physiological variation, and/or other predefined parameters. The output of the predictive model 412a may be the dynamic baseline information 412b of the physiological state of the subject.

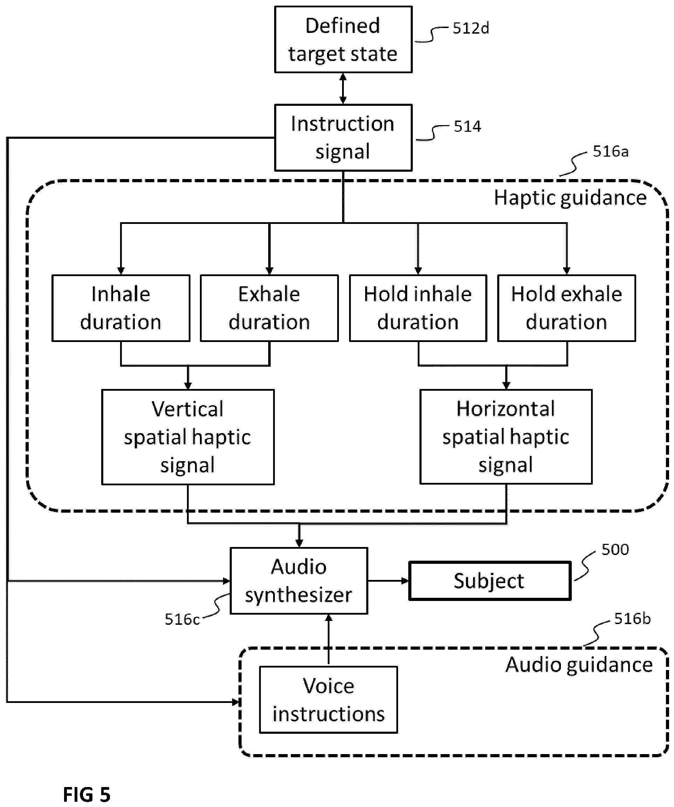

Figure 5 shows how breathing instructions are modulated by a biofeedback signal. Biofeedback based on defined target state 512d and instruction signal 514 may be divided into haptic guidance 516a and audio guidance 516b. Haptic guidance 516a may include a vertical spatial haptic signal that instructs inhale and/or exhale duration. Haptic guidance 516a may include a horizontal spatial haptic signal that instructs holding of inhale breath duration and/or holding of exhale breath duration. Audio guidance 516b may include voice instructions. Haptic guidance 516a and audio guidance 516b may be delivered to the subject 100 300, 400, 500 via an audio synthesizer 516c.

## Claims

1. A biofeedback system comprising
- biosignal acquisition unit (102) configured to measure at least one biosignal from a subject (100, 300, 400, 500),
- pre-processing unit (104), to remove artifacts, and/or increase the signal-to-noise ratio of the at least one biosignal,
- a processing unit (110) comprising
- a storage for at least one database,
- a biographical and historical database (108, 408) for subject data including at least the at least one biosignal, and
- at least one processor,
wherein the at least one processor is configured to
- extract features (130, 330, 430) from the at least one biosignal, wherein at least one autonomous nervous system parameter (402) is extracted,
- estimation of an individual physiological state (112) of the subject (100, 300, 400, 500) based on
- the feature extraction (130, 330, 430),
- the biographical and historical database (108, 408) based dynamic baseline information (412b) calculated using a predictive model (412a), and
- stress level estimation (412c) calculated from the at least one autonomic nervous system parameter and the dynamic baseline information (412b),
- defined target state (412d) calculated from stress level estimation (412c), dynamic baseline information (412b) and subject (100, 300, 400, 500) input, calculation of an instruction signal (114, 414, 514) based on the stress level estimation (412c) of the individual physiological state (112), and
- determination of a guidance signal (116, 416, 516abc) based on the instruction signal (114, 414, 514) and the defined target state (412d),
- a biofeedback unit (120) configured to
- deliver to the subject physiological state-dependent biofeedback according to the guidance signal (116, 416, 516abc) based on the calculated instruction signal (114, 414, 514), wherein the biofeedback is arranged for guidance of the direction, depth, and/or frequency of the breathing of the subject (100, 300, 400, 500),
- compare the individual physiological state (112) before the delivered guidance signal (116, 416, 516abc) with the individual physiological state (112) determined after the delivered guidance signal (116, 416, 516abc),
- adjust feedback parameters (420) of the instruction signal (114, 414) and guidance signal (116, 416, 516abc) such that the feedback outcome approaches the defined target state (412d), and
- update the biographical and historical database (108, 408).

2. System according to claim 1, wherein the biosignals are related to the state of the autonomous nervous system of the subject.

3. System according to any of the preceding claims, wherein the instruction signal (114, 414, 514) is the guidance signal (116, 416, 516abc).

4. System according to any of the preceding claims, wherein the biofeedback is arranged for haptic guidance (516a) of vertical spatial haptic signals instructing inhale and exhale duration and horizontal spatial haptic signals instructing inhale and exhale hold duration.

5. A system according to any of the preceding claims, wherein the guidance signal (116, 416) includes complex multichannel audio-haptic stimulation using low frequency sound waves (20-200 Hz), their harmonics, interference patterns, and relative phase-amplitude modulations corresponding to the perceived effect of their slow (2-12 cm/s) spatial propagation in vertical and horizontal directions.

6. System according to any of the preceding claims, wherein the biosignal acquisition unit (102) is a sensor arrangement to receive, amplify, and transmit the at least one biosignal.

7. System according to any of the preceding claims, wherein the biographical and historical database (108, 408) comprises:
- biographical information,
- previous measurement history,
- previous biofeedback history,
- population level-statistics,
- circadian data, and/or
- one or more sources of individual physiological variation.

8. System according to any of the preceding claims, wherein the biofeedback includes audio guidance (516b) as audible audio stimulus or feedback based on the instruction signal (114, 414, 514) and the defined target state (412d).

9. System according to any of the preceding claims, wherein the pre-processing unit (104) is integrated to the processing unit (110).

10. System according to any of the preceding claims, wherein the biofeedback system is arranged into a seating arrangement.

11. System according to claim 10, wherein the contact points between the subject (100, 300, 400, 500) and the biosignal acquisition unit (102) are arranged on armrests of the seating arrangement.

12. System according to claims 10-11, wherein contact points between the subject (100, 300, 400, 500) and the biosignal acquisition unit (102) are arranged to receive
- the biosignals from forearm area of the subject, and
- the reference and grounding from elbow area of both arms of the subject.

13. System according to claims 1-10, wherein contact points between the subject (100, 300, 400, 500) and the biosignal acquisition unit (102) are arranged in a backrest of the seating arrangement.

14. A biofeedback method comprising
- measuring at least one biosignal from a subject (100, 300, 400 500),
- pre-processing (104) the at least one biosignal to remove artifacts, and/or increase the signal-to-noise ratio of the at least one biosignal,
- storing the at least one biosignal,
- arranging a biographical and historical database (108, 408) for the at least one biosignal,
- extracting features (130, 330, 430) from the at least one biosignal,
- estimating an individual physiological state (112) of the subject (100, 300, 400, 500) based on the feature extraction (130, 330, 430) and the biographical and historical database (108, 408),
- defining a target state (412d),
- calculating an instruction signal (114, 414) based on the estimated individual physiological state (112),
- determining a guidance signal (116, 416, 516abc) based on to the instruction signal (114, 414, 514) and the defined target state (412d),
- providing to the subject vibrotactile biofeedback according to the guidance signal (116, 416, 516abc), wherein the biofeedback is arranged for breathing guidance,
- comparing the individual physiological state (112) before the delivered guidance signal (116, 416, 516abc) with the individual physiological state (112) after the delivered guidance signal (116, 416, 516abc), and
- adjusting feedback parameters (420) of the instruction signal (114, 414) and the guidance signal (116, 416, 516abc) such that the feedback outcome approaches the defined target state (412d), and updating the biographical and historical database (108, 408).

15. A method according to claim 14, wherein calculating the instruction signal (114, 414, 514) includes calculating a ratio between the current stress level estimation (412c) of the at least one biosignal, and a dynamic baseline information (412b) based on a predictive model (412a) calculated from the biographical and historical database (108, 408).
